# EUROPEAN PATENT SPECIFICATION

(11) **EP 3 254 725 B1**
(45) Date of publication and mention of the grant of the patent: **25.12.2019**
(21) Application number: 16746828.9
(22) Date of filing: 02.02.2016
(51) Int. Cl.: A61N 1/05, A61N 1/36, A61N 1/02, A61N 1/04, A61N 1/40, A61N 1/06, A61N 1/32

(54) **APPARATUS FOR TREATING BLOOD VESSELS IN SKIN**
VORRICHTUNG ZUR BEHANDLUNG VON BLUTGEFÄSSEN IN DER HAUT
APPAREIL DE TRAITEMENT DE VAISSEAUX SANGUINS DANS LA PEAU

(30) Priority: 03.02.2015 KR 20150016793; 13.08.2015 KR 20150114641
(43) Date of publication of application: 13.12.2017
(73) Proprietor: Na, Jongju, Seoul 05647 (KR)
(72) Inventor: Na, Jongju, Seoul 05647 (KR)
(74) Representative: Walaski, Jan Filip
(86) International application number: PCT/KR2016/001140
(87) International publication number: WO 2016/126087

(56) References cited:
- WO-A1-00/27466
- WO-A1-2010/014259
- WO-A1-2010/016848
- WO-A2-2011/132856
- KR-A- 20130 106 016
- KR-A- 20150 007 938
- US-A1- 2005 222 565
- US-A1- 2012 046 658

## Description

The present invention relates an apparatus for treating vessel in skin or vessel associated with skin appendages in skin by delivering an electric signal. More particularly, it relates to treating vessel by delivering electric signal, such as high frequency or radio waves, into skin via electrodes, which penetrate skin, to elicit a therapeutic thermal response by delivering electric signal, concentrated on or around targeted vessels instead of dermal matrix, thereby preventing injury to surrounding tissue.

While sun exposure, pregnancy, medications, hormonal changes in the body, and genetic factors are thought to affect the development of melasma, dermal melasma, hyperpigmentation, hypopigmentation, rosacea, flushing, erythema, and telangiectasia, the exact mechanisms thereof have yet to be clarified.

Meanwhile, although treatments for hair loss, hair removal, excessive sebaceous gland secretion, excessive sweating, and axillary osmidrosis are increasing in popularity, their efficacy is limited. Research and development of treatment that provide better therapeutic results are warranted.

Conventional skin treatments with LASER and multi-wavelength light therapy, such as intensive pulsed light (IPL), are not only ineffective at treating targeted vessels at deeper portions of skin, but they also pose an increased risk of damaging skin due to excessive heating thereof.

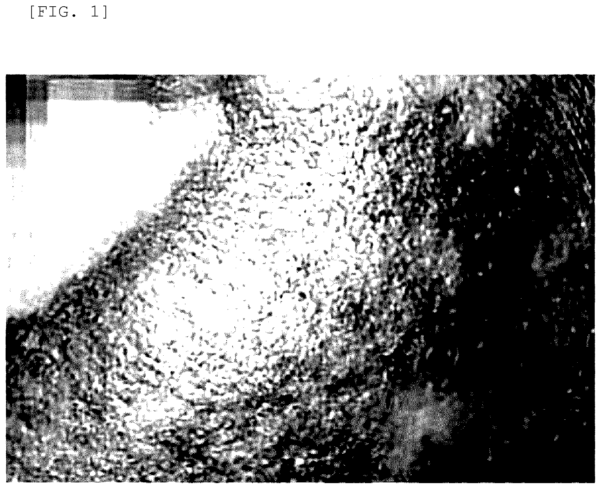

Of these conventional therapies, IPL is often associated with skin burn and post-inflammatory hyperpigmentation (PIH), as shown in Figure 1.

WO2010016848 discloses a system and method used in treating dermalogical tissue. WO2010014259 discloses neurostimulation assemblies, systems, and methods. WO0027466 discloses an electrical stimulation apparatus for delivering an electrical field over a predetermined period of time to a targeted body tissue in order to stimulate a cell initiated angiogenic response in living cells within the targeted body tissue. KR20130106016 discloses an apparatus for skin care using La-effect comprising a needle fixing unit, a driving unit, an electric energy transferring unit, a cooling plate, a heat transfer means, and a heat exchange means.

The invention is defined in the appended claims. Aspects, embodiments and examples disclosed herein which do not fall within the scope of the appended claims do not form part of the invention, and are merely provided for illustrative purposes.

It is an aspect of the invention to provide an apparatus for treating a vessel in skin as set out in claim 1.

The objective of the present invention is to offer a therapeutic apparatus with which to reduce hypervascularity in dermal or epidermal skin layers by generating thermal damage thereto or inducing phagocytosis or apoptosis of vascular cells in order to treat melasma, dermal melasma, hyperpigmentation, hypopigmentation, rosacea, flushing, erythema, or telangiectasia, thereby lowering the risk of excessive heating of skin encountered in and reducing the rate of re-occurrence associated with conventional LASER treatments for melasma, dermal melasma, hyperpigmentation, hypopigmentation, rosacea, flushing, erythema, or telangiectasia.

The present invention not only allows its users to normalize hypervascularity in the dermis or epidermis by generating thermal damage thereto or inducing phagocytosis or apoptosis of vascular cell in order to treat melasma, dermal melasma, hyperpigmentation, hypopigmentation, rosacea, flushing, erythema, or telangiectasia, but also to accurately control degrees of thermal injury delivered to vessel directly through electrodes inserted in the skin. Applying heat selectively to vessel in the skin, instead of applying heat to the entire skin, reduces the adverse effects caused by overheating of the skin, such as burns, which are common in conventional treatments for melasma, dermal melasma, hyperpigmentation, hypopigmentation, rosacea, flushing, erythema, or telangiectasia. Eliminating vessel in the skin also lowers the rate of re-occurrence associated with conventional treatments for melasma, dermal melasma, hyperpigmentation, hypopigmentation, rosacea, flushing, erythema, or telangiectasia.

### BRIEF DESCRIPTION OF THE DRAWINGS

FIG. 1 depicts the side effects of conventional treatment devices.
FIG. 2 is a simple diagram of one embodiment of the present invention for generating electrical signal to treat vessel in skin.
FIG. 3 illustrates the principle mechanism of vessel treatment using an embodiment of the present invention to deliver electrical signal on vessel in skin.
FIGS. 4 to 12 depict the results of animal experiments and the clinical effects of an apparatus for delivering an electrical signal to vessels in skin according to one embodiment of the present invention.
FIG. 13 is a diagram of one waveform of an electrical signal generated by an embodiment of the present invention.

### VARIOUS EMBODIMENTS FOR IMPLEMENTING THE INVENTION

Hereinafter, the present invention will be described in detail with reference to the drawings. Like elements in the drawings are represented by the same reference numerals as possible. In the following description, well-known functions or superfluous explanation about configurations are not described in detail since they would obscure the subject matter of the present invention.

According to recent studies conducted by the present inventors, lesions of melasma, dermal melasma, hyperpigmentation, hypopigmentation, rosacea, flushing, erythema, or telangiectasia, as well as hair loss, hair removal, excessive sebaceous gland secretion, excessive sweating, and axillary osmidrosis, are related to the number, size, shape, and function of vessels therein.

For example, melasma, pigmentation, rosacea, flushing, and telangiectasia lesions clinically and histologically show significant increases in the number and size of blood vessels in the dermis or epidermis, compared to nearby normal skin.

And altering blood vessels in these lesions according to treatment purpose has been found to help to treat states thereof.

Hair loss can be treated by increasing blood circulation to hair follicles lacking blood supply, while excessive sebaceous gland secretion and excessive sweating can be treated by diminishing abnormal increases in the number and size of vascular structures to target glands.

While rosacea, flushing, erythema, and telangiectasia are generally known to be symptoms of underlying vascular problems, the inventor of the present invention also finds that melasma, dermal melasma, hyperpigmentation, and hypopigmentation lesions are closely related thereto.

Moreover, we have noted excessive development or deterioration of blood vessels that supply nutrition to structures in skin important to hair loss, hair removal, excessive sebaceous gland secretion, excessive sweating, and axillary osmidrosis.

Accordingly, the present invention has been developed to transmit an electrical signal to skin through invasive electrodes for selective treatment of blood vessels, which have a particularly higher impedance relative to surrounding skin tissue: selective treatment of tissue in skin is possible due to differences in tissue impedance, conductivity, and dielectric permittivity between individual layers of skin tissue and appendages.

The present invention is capable of controlling intensity of electric signals that are to be emitted to vessels in order to control degrees of thermal reactions induced on vessels. This may allow a user to selectively achieve a desired effect among congestion, regeneration, remodeling, growth, regrowth, degradation, or degeneration of vascular structures in skin.

Thus, the present invention is an apparatus for treating melasma, dermal melasma, hyperpigmentation, hypopigmentation, rosacea, flushing, erythema, telangiectasia, hair loss, or hair removal with vessel treatment.

Additionally, the present invention may be used to treat excessive sebaceous gland secretion (hyperseborrhea), excessive sweating (hyperhidrosis), or related disorders, such as axillary osmidrosis.

The present invention may allow effective treatment of hyper- or hypovascularity in the dermis, epidermis, or skin appendages by generating an optimal thermal effect to target vessels therein or by inducing phagocytosis or apoptosis of target vascular cells. The present invention may prevent side effects of too much heat being applied to vessels or reduce recurrence rates of lesions caused by rebounded hyperplasia of removed vessels after conventional treatments.

The present invention may provide an apparatus for treating melanocytes or basement membranes in skin with improved pathological structure or function, or an apparatus for affecting amounts or function of vascular endothelial growth factor (VEGF), which is derived from vessels.

Meanwhile, conventional invasive high-frequency devices are limited to coagulating skin with heat for primary purpose of neocollagenesis, hemostasis, and cauterizing vessel directly with high heat.

Unlike conventional devices, the present invention encompasses an apparatus for treating vessel in skin by forming electric field within skin. More specifically, the present invention relates to technology of forming electric field via two or more electrodes that penetrate skin.

According to my studies, the present invention has been developed such that, when an electric field is uniformly formed in skin, an electrical signal emitted therein induces a thermal effect on blood vessels first rather than surrounding skin tissue. Thus, unlike the direct electrocauterization of blood vessels, an electric signal of a uniformed electric field facilitates selective heating of blood vessels in treatment of various lesions caused by abnormal vascularity.

While conventional invasive high-frequency devices aimed at stimulating collagen production in skin require the application of a relatively long conduction time, the present invention utilizes with a much shorter conduction time. Moreover, in addition to conduction time, other treatment parameters, such as voltage, power, impedance in skin, etc., can also be controlled, as appropriate.

In addition, due to their high conductivity, blood vessels may be coagulated first with the present invention before the epidermis or dermis is coagulated.

With the present invention, the shortest conduction time at which selective coagulation of blood vessel occurs may be usually within 50 msec, more generally within 100 msec, or even up to within 300 msec.

In order to prevent excessive thermal damage to tissue, except targeted vessels, or to concentrate the desired thermal effect on the vessel, an electrical signal may have one or more pulsed (repeated) conduction times with a delay time (for example, 5-100 msec).

By applying a repetitive electrical signal with a delay time, the applied voltage can be increased, allowing for a greater distance between electrodes (153). Also, the greater voltage can increase the degree of thermal effect delivered selectively on blood vessels over a shorter conduction time, thereby minimizing unwanted injury to surrounding tissue.

In other words, in the present invention, delay time can allow for a greater applied voltage, which induces a faster reaction from blood vessels, preventing thermal injury to surrounding tissue that would likely occur from sustained delivery of electrical energy.

With the present invention, a conduction time of a repetitive electrical signal can be varied according to configuration of individual parameters of the apparatus, in which conduction time can be set to be the same or different in each cycle during an electrical signal. Also, the length of delay time can be varied in the same manner.

Additionally, the conduction time in the present invention can be varied depending on the set voltage, electric power, and electric current of electrical signal, as well as the size, thickness, and quantity of target vessels in skin. The insertion depth, thickness of electrode, distance between electrodes, and deployment of electrodes can also vary the conduction time.

The electrode described in the present invention can be arranged to deliver an electrical signal in bipolar configuration. The present invention can be applicable in all cases of vessel treatment, except for direct electrocautery of blood vessel.

Melasma, dermal melasma, pigmentation, rosacea, flushing, or telangiectasia lesions clinically and histologically show significant increases in the number and size of blood vessels in dermis or epidermis, compared to normal peripheral skin.

The vascular changes apparent in melasma, dermal melasma, pigmentation, rosacea, flushing, or telangiectasia may be due to angiogenesis or vasodilation in association with VEGF (Vascular Endothelial Growth Factor) production.

In melasma, dermal melasma, pigmentation, rosacea, flushing, or telangiectasia, abnormal vascularity in the lesion may act as the main reason for the incidence or aggravation thereof, because the number of blood vessels distributed throughout the lesions has been shown to be significantly related to the amount of pigment therein.

Accordingly, the present invention provides a treatment device which can reduce target vessels including neovascularization, vessel dilation, or hypervascularity that cause the above conditions.

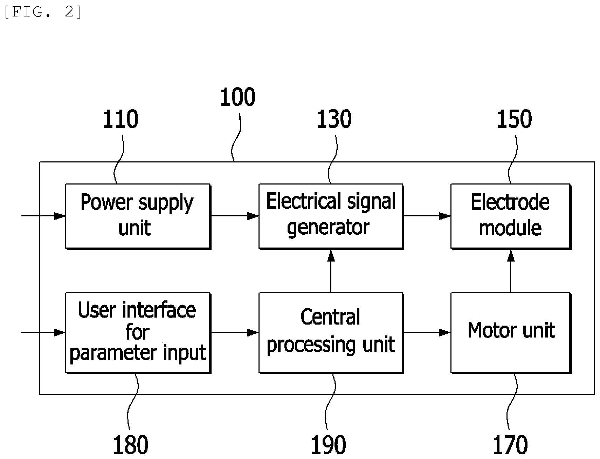

FIG. 2 is a simple diagram of an embodiment of the present invention for delivering electrical signals to treat vessels in skin. As seen in FIG. 2, the treatment apparatus (100) for generating an electrical signal with which to treat vessels in skin, according to an embodiment of the present invention, can include a power supply unit (110), an electrical signal generator (130), an electrode module (150), a motor unit (170), a user interface for parameter input (180), or a central processing unit (190).

The electrode module (150) can include a fixed portion (151) to which two or more electrodes (153) are attached. The power supply unit (110) can supply power from outside the system to the electrical signal generator (130). The electrical signal generator (130) can deliver an electrical signal to the electrode (153) of the electrode module (150) via an electrically conductive material, such as an electrical signal transmission line (135) .

In the present invention, the electrode (153) may also be connectable directly or indirectly with the electrical signal generator (130) or the motor unit (170), omitting the electrode module (150).

In addition, in the present invention, either the electrode (153) or the electrode module (150) may be directly or indirectly connectable electrically to the electrical signal generator (130).

In the present invention, the electrode (153) may be needle shaped and composed of a conductive material.

The electrical signal generated by the electrical signal generator (130) is an electromagnetic wave, with a frequency ranging from 300 Hz to 300 GHz.

In the present invention, the electrical signal generator (130) may generate an intermediate frequency, a high frequency, a radio frequency, an electromagnetic wave, or an ultrasonic wave.

The electrical signal generated by the electrical signal generator (130) may be emitted in the form of an electromagnetic wave of a predetermined frequency. The electrical signal may be transferred to two or more electrodes (153). Heat is then generated on or around a target vessel (300) by transmission of the electrical signal into the lesioned area of the skin (200) via the electrode (153).

During use of the present invention, the operator may insert the electrode (153) directly into the lesioned skin (200) by hand, or the electrode (153) may be attached to the electrode module (150) for insertion.

The present invention may include a motor unit (170) that automatically drives the electrode (153) or the electrode module (150) to which the electrode (153) is inserted to a predetermined depth into the skin. The motor unit (170) may be connected directly or indirectly by other intermediates to the electrode (153) or electrode module (150).

In addition, the electrode (153) or the electrode module (150) may not be connected to the motor unit (170) before insertion into skin. It may be inserted into skin by pressing on the electrode (153) or the electrode module (150) by the motor unit (170) during the electrode penetration phase.

The motor unit (170) may transmit directly or indirectly the force that drives to penetrate two or more electrodes (153) into the skin (200) at a predetermined depth (approximately 1 mm to 2.5 mm).

With the present invention, two or more electrodes (153) may be inserted into the skin (200) penetrating the epidermal layer (210) at a predetermined depth of approximately 0.2 mm to 1 mm or the dermal layer (220) at a predetermined depth of approximately 1 mm to 4 mm. The insertion depth of the electrode (153) into target skin (200) may range from 0.2 mm to 4 mm.

However, it may also be possible to treat a target vessel by placing the electrode (153) on the surface of the skin (200) without penetrating into skin (200) .

As described above, the depth of the electrode (153) insertion into target skin (200) may be within 4 mm. However, the depth of insertion of the electrode (153) into the skin (200) may be deeper than 4 mm in targeted areas where skin thickness is relatively thicker and could correspond to the entire thickness of the skin (200) layer.

In the present invention, various means capable of moving the electrode (153) or the electrode module (150) linearly can be used as the motor unit (170), such as an actuator, a motor, a linear motor, a stepping motor, an electromagnet, or a piezoelectric element, etc.

The user interface (180) may include the magnitude of the voltage, the amount of electrical current, the resistance value, the impedance of the tissue, or the electrical conduction time applied to the electrode (153) from the electrical signal generator (130). Additionally, the depth of electrode insertion into the skin may be included.

Upon receiving a control command from the user interface (180), the central processing unit (190) may signal the electrical signal generator (130) to deliver the desired magnitude of voltage that is to be applied to two or more electrodes (153), the amount of electrical current, the resistance value, the impedance, or the electrical conduction time to control the energy amount of the electrical signal.

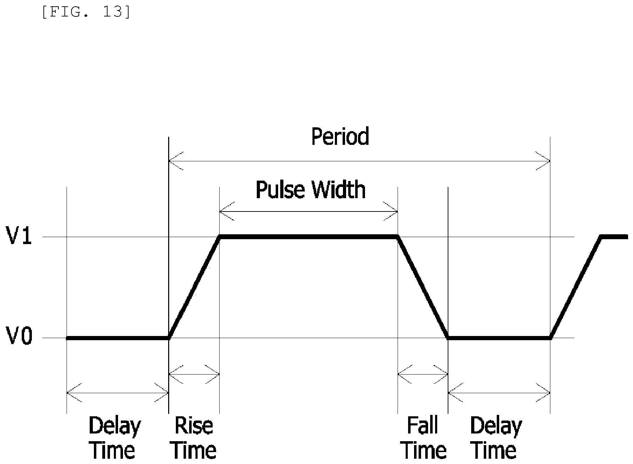

Additionally, the central processing unit (190) may control the power supply unit (110) such that it repeatedly supplies power to the electrical signal generator (130) over a predetermined time interval. As shown in FIG. 13, the central processing unit (190) may control the electrical signal generator (130) such that it generates an electrical signal repeatedly at a predetermined time interval.

During use of the present invention, the applied voltage (V1) may be in the range of 10 volts to 400 volts (preferably 20 to 300 volts), based on a 100-ohm load. A delay time may be in the range of 0.1 msec (millisecond) to 500 msec (preferably 5 to 300 msec). A conduction time may be in the range of 1 msec to 450 msec (preferably 5 to 300 msec).

If the delay time is too short (less than 0.1 msec), thermal damage will occur to tissues other than the target vessel (300), whereas too long of a delay time (longer than 500 msec) would be too long to induce enough thermal response on the target vessel (300). Conversely, if the conduction time is too long (greater than 450 msec), excessive heat may be generated on tissues other than the target vessel (300), and if the conduction time is too short (less than 1 msec), the thermal response elicited on the target vessel (300) may be insufficient.

The number of repeated cycles of the electrical signal conduction and the delay time may be closely related to the applied voltage, electric power, conduction time, or delay time etc. Delivery of too few a number of repeated cycles may fail to generate a sufficient thermal response, whereas too many may cause excessive thermal damage to other tissues around the vessel (300).

The present inventor have confirmed experimentally that a more preferable therapeutic effect is achieved with application of a relatively high output voltage over repeated cycles of electrical signal conduction with a delay time than with application of a low output voltage over continuous electrical signal conduction without a delay time.

When the present invention is utilized, as shown in FIG. 13, the electrical signal generator (130) may generate a pulse type electrical signal, wherein a pulse of alternating current (A.C.) is preferable to a pulse of direct current (D.C.) in regards to generating a thermal response.

With the present invention, when inserting two electrodes into the skin around a target vessel (300), the electrode (153) may be implemented in a bipolar configuration. An Alternating Current Pulsed-typed Electric Field may be formed in the skin, upon applying an A.C. pulse of electrical signal via the electrode (153) inserted into skin.

Specifically, high-frequency pulse signal of A.C. polarity may be much more effective at vibrating water molecules in skin than those of D.C. polarity and, thereby, generating a greater thermal response on target vessels (300).

In the present invention, the alternating polarity of high-frequency pulse signal of A.C. polarity may allow for generating selective thermal response to target vessel (300), compared with high-frequency pulse signal of D.C. polarity.

Accordingly, in the present invention, the electric field (250) formed between two electrodes (153) inserted around a target vessel (300) may preferably be an A.C. electric field.

Also, when a high output voltage is applied, a uniform and strong electric field can be formed in a wider area, compared to when a low output voltage is applied. However, since the thermal response elicited on a target vessel (300) occurs more rapidly in the electric field of high output voltage, it is necessary to shorten the conduction time.

With the present invention, the user can set the depth of insertion of the electrode (153) into the skin via the user interface (180), and accordingly, the central processing unit (190) may control the degree to which the motor (170) moves the electrode (153).

Thus, via the user interface (180), the user may input at least one parameter among voltage, power, and conduction time of the electrical signal and may set the insertion depth for the electrode (153). The central processing unit (190) then may control the electrical signal generator (130) to generate the predetermined electrical signal, which may be converted from A.C. or D.C. from the power supply unit (110), into a predetermined electric signal by the electrical signal generator (130). The electrical signal is then transmitted to the electrode (153).

The central processing unit (190) may control the motor unit (170) to drive individual electrode (153) or the electrode module (150) into the skin (200) according to the electrode (153) insertion depth set by the user through the user interface (180).

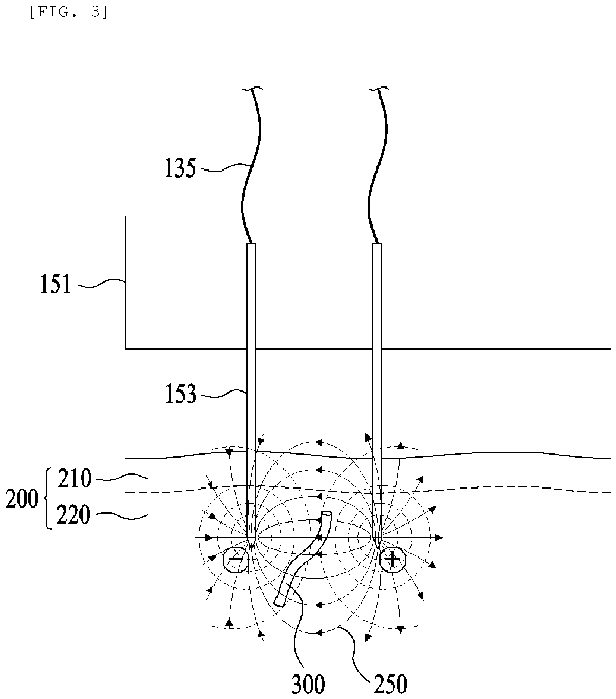

FIG. 3 illustrates the principle mechanism of vessel treatment using an embodiment of the present invention to deliver electrical signal to vessel in skin.

As drawn in FIG. 3, the skin (200) comprises the epidermal layer (210) and the dermal layer (220). The dermal layer (220) is primarily where target vessel (300) that give rise to melasma, dermal melasma, pigmentation lesion, rosacea, flushing, telangiectasia, etc. are located.

In some cases, however, a target vessel (300) may be distributed in the epidermal layer (210), while in others, a target vessel (300) may be distributed throughout both the epidermal layer (210) and the dermal layer (220).

With the present invention, the electrode (153) can be inserted within the epidermal layer (210) only or further into the dermal layer (220) as needed, depending on the distribution of a target vessel (300) throughout the skin.

Herein, a vessel that gives rise to melasma, dermal melasma, hyperpigmentation, hypopigmentation, rosacea, flushing, erythema, or telangiectasia lesions is referred to as a target vessel (300).

As shown in FIG. 3, when a target vessel (300) is present only in the dermal layer (220), it is preferable that the inserted electrode (153) penetrates the dermal layer (220). However, the electrode (153) may be inserted such that it penetrates only the epidermal layer (210) with appropriate energy settings (voltage, power, and conduction time).

Alternatively, it may also be possible to generate a sufficient thermal response with which to treat a target vessel (300) by placing the electrode (153) on the surface of the skin without penetrating the skin.

For treating melasma, dermal melasma, pigmentation, rosacea, flushing, or telangiectasia lesions in the dermal layer (220), the electrode (153) into the dermal layer (220) may be inserted among target vessels (300) within the lesioned area of the skin.

Additionally, electrode (153) may be inserted into the skin with a target vessel (300) positioned between two electrodes (153). Upon doing so, an electric field (250), as shown in FIG. 3, may be formed between the two electrodes (153).

Alternatively, a target vessel (300) may still experience a thermal response if located deeper than the electrode (153) can penetrate or if positioned immediately beneath the electrode (153), as long as the target vessel (300) is located within the electric field (250) formed between the electrodes (153).

It is preferable that electrodes (153) are inserted into the skin with a target vessel (300) positioned in the A.C. electric field (250) formed between two electrodes (153).

Thereby, with the present invention, conducting a high-frequency signal of A.C. polarity to the electrode (153) may induce a selective thermal response on a target vessel (300) positioned between two bipolar electrodes (153) .

By inducing a selective thermal response confined to a target vessel (300) using the A.C. electric field (250), selective treatment on vessels that spares injury to surrounding tissue is possible.

When using the present invention, it may be preferable that the high-frequency signal applied to the electrode (153) has a frequency between 0.1 MHz and 100 MHz.

In varying instances, sometimes a target vessel (300) may be positioned adjacent to the electrode (153); other times they may be relatively far from the electrode (153). When using the present invention, the user can change the type of electrical signal or adjust the voltage, power, or conduction time of the desired electrical signal according to the distance from the electrode (153) to a target vessel (300).

The electrode (153) inserted into the skin (200) receives an electrical signal via the electrical signal transmission line (135), and an electrical signal transmitted to the inserted portion of the electrode (153) is emitted on the blood vessel (300).

The electrical signal transmission line (135) may be connected directly to the electrode (153) or indirectly via a printed circuit board, a solder, an electrical pin (a pin that is capable of conducting electric power and being bent and stretched), a pogo pin, an electric conduction plate, an electric conduction rod, or an electrical connector to transmit an electrical signal.

The therapeutic mechanism by which an electrical signal induces thermal injury to a target vessel (300) is briefly described. With the present invention, an electrical signal in the lesion may be concentrated to a target vessel (300) in the skin, generating heat in the target region to treat the target vessel (300).

The present treatment apparatus (100) can be designed to deliver an electrical signal to the electrode (153) inserted into the skin or in contact with the skin surface in order to generate thermal injury on or around a target vessel (300) in the skin.

Through clinical experiments on the treatment apparatus (100) for generating an electrical signal with which to treat vessels in the skin, the inventor thereof has discovered the following:

The causative target vessel (300) that give rise to lesion, such as melasma, dermal melasma, pigmentation, rosacea, flushing, or telangiectasia, are mostly neovascular (premature blood vessel), and the binding between the cells that constitute their vessel walls is in a loose state, relative to normal vessels. Moreover, the thickness of the vessel wall for neovasculature is thinner than that for normal blood vessels, and the cellular structures in the vessel wall are weak. Thus, unlike normal blood vessels, these immature blood vessels can be easily destroyed by relatively weak electrical stimulation.

Accordingly, by applying electrical signal to destroy the causative, a target vessel (300) that gives rise to lesions, such as melasma, dermal melasma, pigmentation, rosacea, flushing, or telangiectasia, treatment for these conditions may be possible, although it is not simple.

To induce suitable damage on a target vessel (300), an electrical signal that is neither too weak nor too strong is required, and therefore, delicate control of the electrical signal generator (130) is required. Since a target vessel (300) of neovascularity treated with a relatively weak signal or with a strong electrical signal results in artificial stimulation, cauterization, elimination, or severe destruction, compensation mechanisms quickly give rise to more new vessels due to a phenomenon known as vascular hyperplasia.

Therefore, treatment methods aimed at simply destroying the causative, target vessel (300) in melasma, dermal melasma, pigmentation, rosacea, flushing, or telangiectasia lesions via an electrical signal of random strength typically result in treatment outcomes of vascular hyperplasia or post-inflammatory hyperpigmentation (PIH), a condition that further exacerbates lesions of melasma, dermal melasma, pigmentation, rosacea, flushing, or telangiectasia.

The changes in lesioned skin elicited by delivering an electrical signal to a vessel in the skin using the present invention will now be described in more detail.

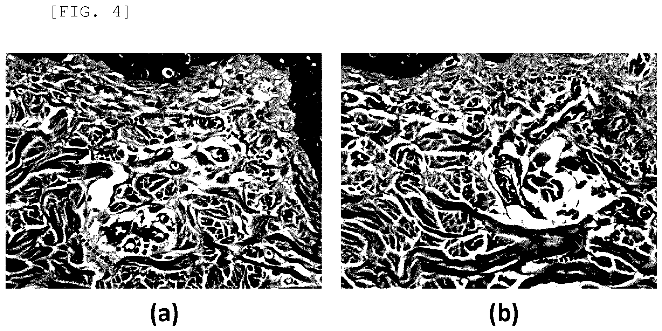

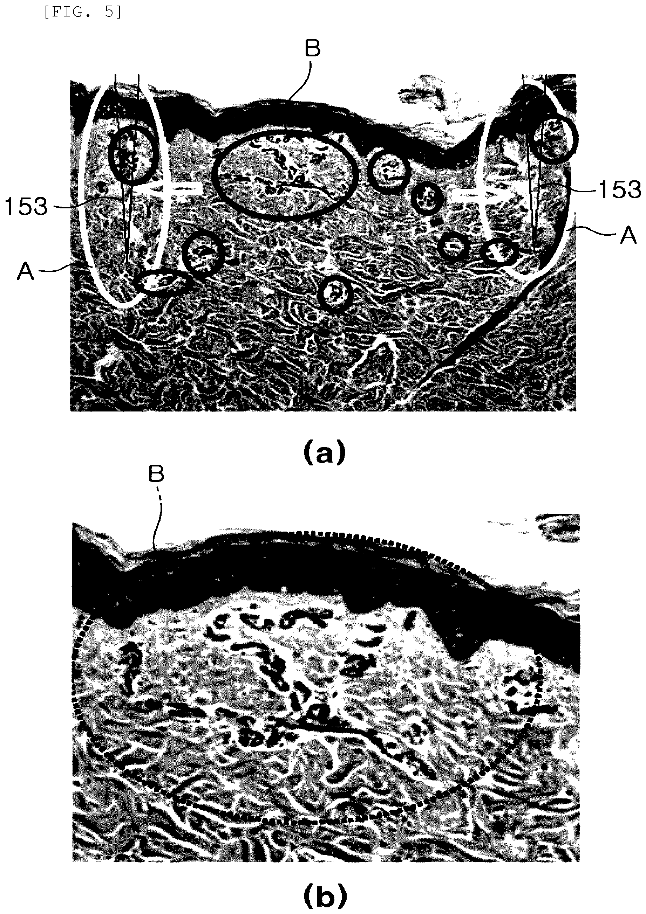

As shown in FIGS. 4 and 5, when the therapeutic apparatus (100) of the present invention was used to apply an electric signal to the skin of a micro pig, vessels in the dermal layer of skin responded selectively to the electric signal. No evidence that the blood vessels were destroyed or that excessive bleeding occurred was recorded.

In FIG. 5(a), heat-damaged tissue (pale pink in color) is observed in regions demarcated by white ovals labelled region A, around which electrodes (153) have been inserted. The black circles labelled as region B highlight areas of selective thermal damage to the walls of vessels (pale pink in color) neighboring the target vessel (300) (the largest black circle).

FIG. 5 (b) is an enlarged photograph of the target vessel (300) in region B of FIG. 5(a). The photograph more clearly exhibits a selective thermal response along the vessel wall (pale pink color).

Note that no thermal damage occurs in skin tissue between the two electrodes (153) in regions A (area around the electrodes), except for area surrounding the target vessel (300) in region B.

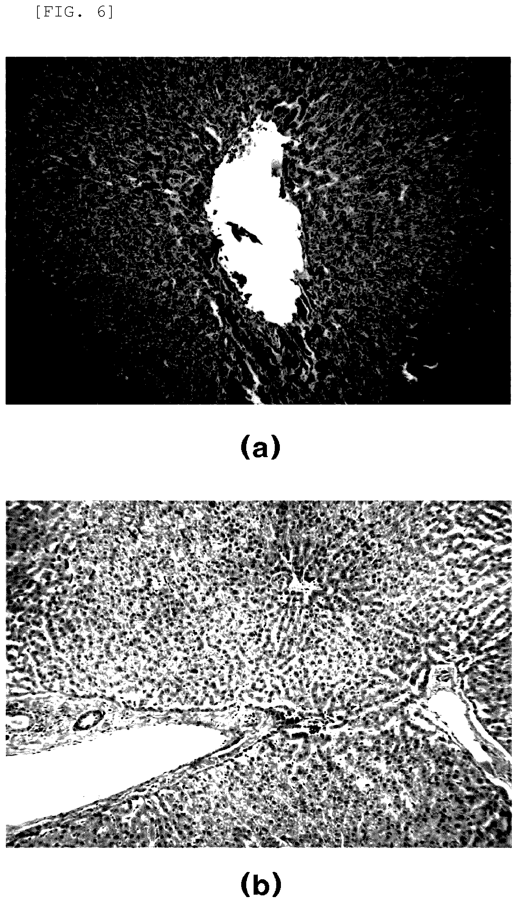

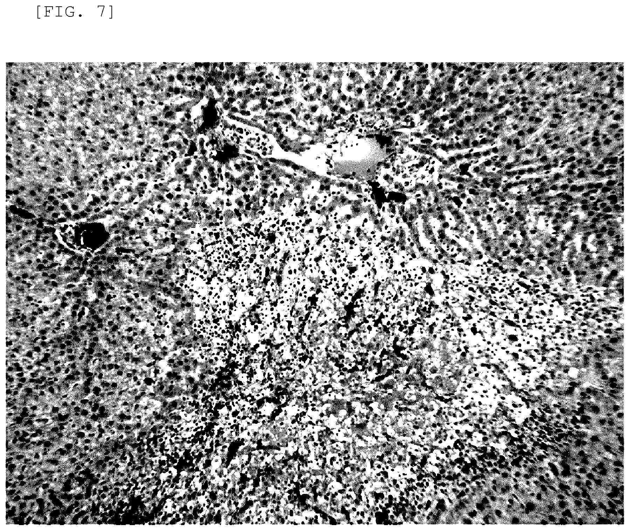

FIGS. 6 and 7 shows experimental results from treating bovine liver tissue with the treatment apparatus (100). Liver tissue was selected in order to more clearly show the selective thermal response on blood vessels, since liver tissue, in comparison with the skin, is composed primarily of hepatocytes and blood vessels and has relatively uniform tissue impedance, conductivity, and permittivity. Doing so confirmed that the present invention delivers an electrical signal that selectively reacts with vessels in bovine tissue.

In addition, the electrical signal mainly induced a thermal reaction along the outer surface of the vessels. Note that the electrical signals are conducted along the vessel wall.

The tissue changes elicited by the thermal reaction induced by an electrical signal were mainly observed in the tunica adventitia of blood vessels, while the tunica intima and tunica media were preserved.

As stated above, non-selective, aggressive destruction of vessels in a lesion stimulates excessive production of Vascular Endothelial Growth Factor (VEGF) and promotes blood vessel regeneration by Vascular Hyperplasia, leading to worsening of the lesion.

However, a selective thermal reaction, such as that induced with the present invention, along a target vessel (300) or the outer layer of a target vessel (300), promotes the regeneration of the target vessel (300) in lesioned skin into normal vessel structure. Moreover, inducing a selective thermal reaction decreases the risk of side effects that may occur as the results of excessive nonselective damage to vascular and dermal structures, and it improves the clinical appearance of melasma, dermal melasma, pigmentation, rosacea, flushing, or telangiectasia lesions.

In clinical experiments on the treatment apparatus (100) for generating an electrical signal with which to treat vessels in skin, the inventor found that, in treatment of melasma, dermal melasma, pigmentation, rosacea, flushing, or telangiectasia lesions, appropriate thermal damage to endothelial cells in vessel walls restores abnormal vascular hyperplasia to normal or normalizes increased VEGF amounts or levels, thereby promoting angiogenesis, and returning dilated vessel to normal size.

Appropriate thermal damage to endothelial cells also can induce the phagocytosis or apoptosis thereof, enhancing the therapeutic effect vessel treatment.

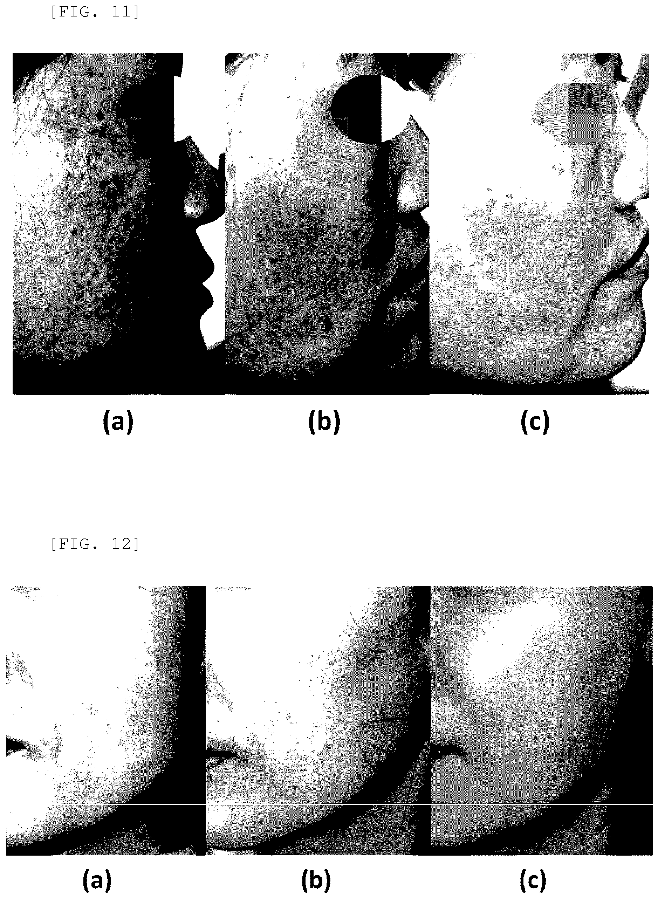

As shown in FIGS. 11 and 12, a vessel removed by the mechanism of inducing voluntary phagocytosis or apoptosis of endothelial cells shows a markedly lower rate of recurrence, which was confirmed in clinical study.

The technical principle of generating appropriate thermal damage to endothelial cells that constitute a causative, target vessel (300) that gives rise to melasma, dermal melasma, pigmentation, rosacea, flushing, or telangiectasia lesions with the present invention will now be described in more detail.

Most of the blood in a target vessel (300) that cause melasma, dermal melasma, pigmentation, rosacea, flushing, or telangiectasia is composed of water, such that the electrical conductivity of a vessel in an electric field is higher than that of surrounding tissue. Thus, vessels strongly attract most of the electrical current delivered to the skin. Also, vessel walls exhibit a much higher difference of impedance and permittivity from blood, and thus, in the presence of an electric field, the ionics that in the vessel wall vibrate, causing a thermal reaction: an electrical signal in the form of an electromagnetic wave cause water molecules and ionics to vibrate, thereby generating friction and heat.

Further, electric signals exit vessels through blood that is in contact with the inner vessel wall, and heat is dispersed by blood flowing through the vessel. Thus, a thermal reaction is less likely to occur inside the vessel wall. Instead, heat is concentrated on the outer vessel wall.

If the conduction time of the electrical signal is further increased, more heat is generated as a whole, although this can cause excessive damage to the blood vessel.

Moreover, the heat generated in the vessel wall causes heat damage to endothelial cells.

In accordance with the above, the treatment apparatus (100) is capable of eliciting a therapeutic thermal response concentrated on and around the vessel wall of a causative, target vessel (300) that gives rise to melasma, dermal melasma, pigmentation, rosacea, flushing, or telangiectasia lesions, without causing injury to surrounding tissue.

Accordingly, by generating thermal damage to vessel in the skin with an electrical signal, the treatment apparatus (100) is a device that can induce selective thermal injury to endothelial cells of a target vessel (300) and can generate frictional heat stemming from the vibration of water and ionic substances (or electrolytes) in a target vessel (300).

The treatment apparatus (100) can be used for normalizing abnormal hyperplasia of endothelial cells or for normalizing increases in VEGF in treatment of conditions exhibiting angiogenesis or vasodilation.

As is stated above, the treatment apparatus (100) also induces phagocytosis or apoptosis of endothelial cells to achieve a therapeutic effect.

In order to induce phagocytosis or apoptosis of endothelial cells, an appropriate degree of thermal injury should be applied to endothelial cells. The results of repeated clinical experiments to find the optimal conditions for generating a sufficient thermal response using the treatment apparatus (100) are shown in Table 1.

**[Table 1]**

| | Condition 1 | Condition 2 | Condition 3 |
|---|---|---|---|
| Voltage (Vrms: 100Ω load) | 5∼400 | 10∼300 | 20∼250 |
| Power (W: 100Ω load) | 0.25∼1,600 | 1.0∼900 | 4.0∼625 |
| Conduction time (msec) | 1∼500 | 5∼400 | 10∼300 |

The voltage and power values in Table 1 are measured values for when the treatment apparatus (100) is applied with a load resistance of 100 Ω (Ohm; resistance).

In other words, the voltage (Vrms unit: Volts) and power values are those at a load resistance of 100Ω (Ohm; resistance) for electrical signal delivered to electrode (153) inserted in the skin (200) (epidermal layer [210] and dermal layer [220]).

The conduction time (msec unit: 0.001 sec) in Table 1 refers to the time over which an electrical signal was applied to the electrode (153) inserted into the skin (200) .

More specifically, the conduction time was measured as the time for delivering an electric signal to the skin (200) during one shot.

When using the present invention, one or more of the parameter values shown in Table 1 can be set in the user interface (180) of the treatment apparatus (100), and the central processing unit (190) can control the delivery of an electrical signal with the treatment apparatus (100) accordingly.

Applying the treatment conditions in Table 1, melasma, dermal melasma, pigmentation, rosacea, flushing, or telangiectasia lesions were improved with condition 1, but showed greater improvement with the narrower treatment conditions in condition 2.

When the treatment conditions were set to condition 3, compared to condition 2, the degree of apoptosis and treatment effects on lesions were more prominent.

Applying the parameters in conditions 2 to 3, the inventor of the present invention conducted clinical tests in humans and animals using the treatment apparatus (100) to treat melasma, dermal melasma, pigmentation, rosacea, flushing, and telangiectasia lesions. The results will now be described with reference to FIGS. 4 to 12.

FIG. 4 presents photographs of results from animal experiments on micro pig skin. Comparing results (a) before treatment and those (b) after treatment, vessel responses to electrical signal were visible in the dermis. There were no signs that the vessels were destroyed or that excessive bleeding occurred. Meanwhile, damage to vascular cells could be confirmed.

FIGS. 6 and 7 are photographs of results from animal experiments on bovine liver tissue immediately after application of electrical signals.

As seen in FIGS. 6 and 7, an electrical signal induced a selective thermal reaction on vessels in bovine liver tissue. The tissue changes elicited by the thermal reaction induced by an electrical signal were mainly observed in the tunica adventitia of blood vessels, while the tunica intima and tunica media layers were preserved.

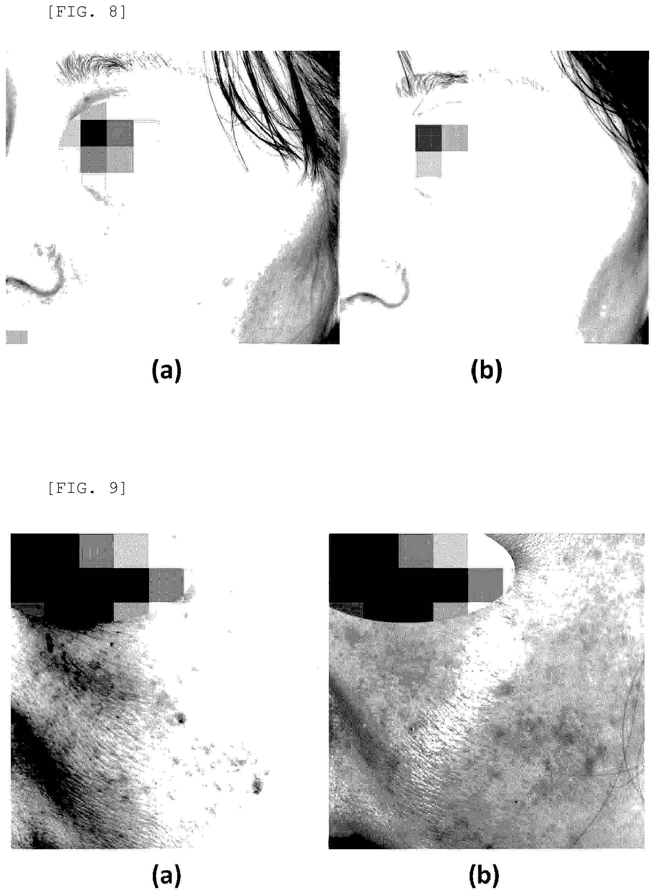

FIG. 8 includes photographs of results from a clinical trial in human skin. Comparing results (a) before treatment and those (b) after 2 months of treatment at 1-week intervals, marked improvement in vascular lesions, such as melasma, dermal melasma, pigmentation, rosacea, flushing, and telangiectasia, was noted.

FIG. 9 includes photographs of results from another patient in the same clinical trial. Comparing results (a) before treatment and those (b) after 2 months of treatment at 1-week intervals, marked improvement in vascular lesions, such as melasma, dermal melasma, pigmentation, rosacea, flushing, and telangiectasia, was noted.

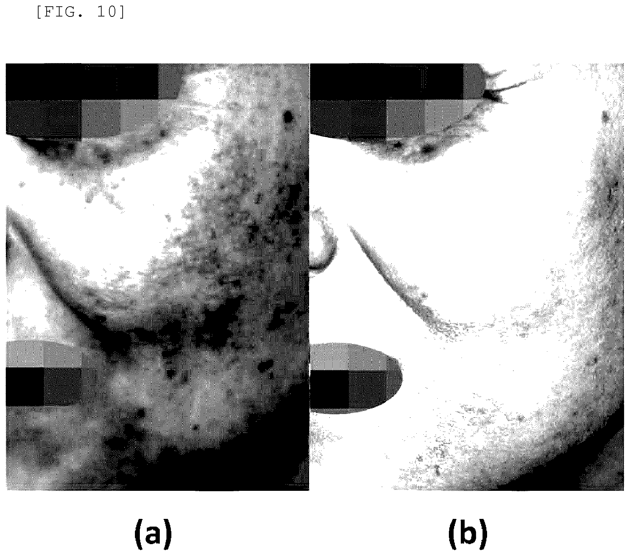

FIG. 10 includes photographs of results from a third patient included in the same clinical trial. Comparing results (a) before treatment and those (b) after 2 months of treatment at 1-week intervals, marked improvement in vascular lesions, such as melasma, dermal melasma, pigmentation, rosacea, flushing, and telangiectasia, was noted.

FIG. 11 includes photographs of results from a fourth patient included in the same clinical trial. Comparing results (a) before treatment and those (b) after 2 months of treatment at 1-week intervals with those (c) at 1 year after completing the treatment course, marked improvement in vascular lesions, such as melasma, dermal melasma, pigmentation, rosacea, flushing, and telangiectasia, was noted. Moreover, unlike conventional treatment, the appearance of the lesion continued to improve until one year after completion of the treatment, and there were no signs of lesion recurrence.

FIG. 12 includes photographs of results from a fifth patient included in the same clinical trial. Comparing results (a) before treatment and those (b) after 2 months of treatment at 1-week intervals with those (c) at 1 year after completing the treatment course, marked improvement in vascular lesions, such as melasma, dermal melasma, pigmentation, rosacea, flushing, and telangiectasia, was noted. Moreover, unlike conventional treatment, the appearance of the lesion continued to improve until one year after completion of the treatment, and there were no signs of lesion recurrence.

With the present invention, electro-thermal responses induced by an electrical signal applied to the skin vary according to the resistance of individual tissues.

During use of the present invention, an electrical signal may be delivered to the skin in a monopolar mode, consisting of an active electrode with negative polarity and a ground electrode with positive polarity, or in a bipolar mode, in which both electrodes are active.

In the monopolar mode, an electrical circuit is formed wherein electric current (electrons) flows from the active electrode through the patient's body to the ground electrode. In the bipolar mode, the flow of the electric current is limited to target tissue. Therefore, the bipolar mode is preferable to the monopolar mode, because the transmission of energy in the bipolar mode is safer to the human body and can be concentrated on target site.

In addition, since the present invention transmits an electric signal to the skin via an electrode that penetrates into target tissue, it can more precisely control the depth of treatment, compared to conventional, non-invasive methods. Moreover, it offers more uniform treatment at deeper regions of the skin. Another advantage is that the discontinuous emission of energy allows for a selective tissue thermal reaction to form in the target tissue.

Additionally, while systemic injury to blood vessels can be fatal, the invasive method facilitates localized treatment that is relatively safe.

In addition, further research with the present invention has indicated that hair roots in the skin are conductors of electric current, particularly the outer sheath, the root muscle, and fibrous connective tissue, similar to the outer walls of vessels.

Therefore, it is expected that the treatment apparatus can help in improving hair loss, which is a complicated lesion. Additionally, by adjusting the intensity of electrical signal applied to the hair follicle, permanent hair removal can be achieved.

In treatment of melasma, the present invention can be performed in combination with conventional treatments, such as LASER toning, drug therapy, etc., in order to increase therapeutic effects and to further lower the risk of recurrence.

In summary, with the present invention, thermal reactions may be selectively induced only on vessel tissue, and unnecessary damage to other surrounding tissues can be prevented.

In addition, by conducting an electrical signal in a pulsed manner, it is possible to avoid unnecessary damage to surrounding tissue and excessive damage to vessel tissue, thereby shortening the recovery period after treatment and reducing the risk of side effects.

In addition, with the present invention, it is possible to control the degree of thermal reaction generated on vessel tissue, thereby preventing bruising, vascular hyperplasia, PIH, etc., which are caused by excessive damage to blood vessels.

Also, in contrast to currently available treatments, the present invention utilizes a penetrating electrode that allows for more uniform treatment of vascular tissue at deeper regions in the skin.

Further, with the present invention, implementation of electrodes in a bipolar configuration confines the transmission of electrical current within target lesions, unlike unipolar electrodes with which electrical current is applied to the whole body. This is particularly advantageous for treating patients suffering from heart disease or who wear a pacemaker, as they would be contraindicated for treatment with monopolar electrodes.

Additionally, with the present invention, as only vascular tissue is selectively treated, pain caused by the thermal reaction can be reduced, providing a more comfortable procedure for the patient.

Use of the present invention for treating blood vessel is not limited to only application in the field of dermatology. The invention may also be applied to treat vessel in tissue of all medical areas, including Gastro Intestinal systems, like the oral cavity, pharynx, larynx, esophagus, stomach, intestines, anus, liver, spleen, gall bladder, or pancreas, or Respiratory systems, like the trachea, lung, pleura, or chest wall, as well as brain, spinal cord, all neurological system and subcutaneous tissues.

The terminology used herein is for the purpose of describing particular embodiment only and is not intended to be limiting of the invention. The singular forms "a," "an," and "the" include plural referents unless the context clearly dictates otherwise. In the present application, the terms "comprises" or "having," etc. are intended to specify the presence of stated features, integers, steps, operations, elements, components, or combinations thereof, but do not preclude the presence or addition of one or more other features, integers, steps, operations, elements, components, or combinations thereof.

The present invention is recognized for its industrial applicability in the medical equipment industry.

## Claims

1. An apparatus for treating a vessel in skin, comprising:
a plurality of electrodes (153) that are inserted in skin to deliver an electrical signal to a target vessel;
an electrical signal generator (130) electrically coupled to the plurality of electrodes (153); and
a power supply unit (110) for supplying power to the electrical signal generator (130),
wherein the target vessel is positioned between two electrodes among the plurality of electrodes (153),
wherein thermal damage is induced to the target vessel by the electrical signal applied to the plurality of electrodes (153),
wherein the electrical signal delivered to the plurality of electrodes (153) is a repetitive electrical signal with at least one delay time, and
**characterized in that** the voltage applied to the plurality of electrodes (153) is in the range of 20 Vrms to 250 Vrms, the power transmitted to the plurality of electrodes (153) is in the range of 4.0 W to 625 W, the duration over which the electrical signal is conducted to the plurality of electrodes (153) is in the range of 10 msec to 300 msec, and the delay time is in the range of 0.1 msec to 500 msec .

2. The apparatus of claim 1, wherein the plurality of electrodes (153) include a bipolar configuration.

3. The apparatus of claim 1, wherein the electrical signal generator (130) is a high-frequency signal generator, which frequency ranges from 0.1 MHz to 100 MHz.

4. The apparatus of claim 1, wherein at least one parameter among a depth of electrode penetration into the target skin, voltage applied to the plurality of electrodes, power transmitted to the plurality of electrodes, duration over which an electrical signal is conducted to the plurality of electrodes, and delay time during which an electrical signal is not conducted to the plurality of electrodes, is set preliminarily.

5. The apparatus of claim 1, wherein the apparatus includes an electrode module (150) comprising an array to which the plurality of electrodes (153) are fixed.

6. The apparatus of claim 5, wherein the apparatus includes a motor unit (170) for driving the electrode or the electrode module to penetrate the electrode into the skin.

## Patentansprüche

1. Vorrichtung zur Behandlung eines Gefäßes in der Haut, umfassend:
mehrere Elektroden (153), die in die Haut eingeführt werden, um ein elektrisches Signal an ein Zielgefäß abzugeben;
einen Generator (130) von elektrischen Signalen, der mit den mehreren Elektroden (153) elektrisch gekoppelt ist; und
eine Energieversorgungseinheit (110) zum Versorgen des Generators (130) von elektrischen Signalen mit Energie,
wobei das Zielgefäß zwischen zwei Elektroden von den mehreren Elektroden (153) positioniert ist,
wobei bei dem Zielgefäß durch das elektrische Signal, das auf die mehreren Elektroden (153) angewendet wird, eine thermische Schädigung induziert wird,
wobei das elektrische Signal, das an die mehreren Elektroden (153) abgegeben wird, ein sich wiederholendes elektrisches Signal mit mindestens einer Verzögerungszeit ist und
**dadurch gekennzeichnet, dass** die Spannung, die an die mehreren Elektroden (153) angelegt wird, im Bereich von 20 Vrms bis 250 Vrms liegt, die Energie, die an die mehreren Elektroden (153) übertragen wird, im Bereich von 4,0 W bis 625 W liegt, die Dauer, über die das elektrische Signal an die mehreren Elektroden (153) geleitet wird, im Bereich von 10 ms bis 300 ms liegt und die Verzögerungszeit im Bereich von 0,1 ms bis 500 ms liegt.

2. Vorrichtung nach Anspruch 1, wobei die mehreren Elektroden (153) eine bipolare Konfiguration beinhalten.

3. Vorrichtung nach Anspruch 1, wobei der Generator (130) von elektrischen Signalen ein Hochfrequenzsignalgenerator ist, wobei diese Frequenz von 0,1 MHz bis 100 MHz reicht.

4. Vorrichtung nach Anspruch 1, wobei mindestens ein Parameter von einer Elektrodeneindringtiefe in die Zielhaut, einer Spannung, die an die mehreren Elektroden angelegt wird, einer Energie, die an die mehreren Elektroden übertragen wird, einer Dauer, über die ein elektrisches Signal an die mehreren Elektroden geleitet wird, und einer Verzögerungszeit, während der ein elektrisches Signal nicht an die mehreren Elektroden geleitet wird, vorher eingestellt wird.

5. Vorrichtung nach Anspruch 1, wobei die Vorrichtung ein Elektrodenmodul (150) beinhaltet, das eine Anordnung umfasst, an der die mehreren Elektroden (153) befestigt sind.

6. Vorrichtung nach Anspruch 5, wobei die Vorrichtung eine Motoreinheit (170) zum Antreiben der Elektrode oder des Elektrodenmoduls zum Eindringenlassen der Elektrode in die Haut umfasst.

## Revendications

1. Appareil de traitement d'un vaisseau dans la peau, comprenant :
une pluralité d'électrodes (153) qui sont introduites dans la peau pour délivrer un signal électrique à un vaisseau cible ;
un générateur de signal électrique (130) couplé électriquement à la pluralité d'électrodes (153) ; et
une unité d'alimentation en énergie (110) pour fournir de l'énergie au générateur de signal électrique (130),
dans lequel le vaisseau cible est positionné entre deux électrodes parmi la pluralité d'électrodes (153),
dans lequel une lésion thermique est induite au vaisseau cible par le signal électrique appliqué à la pluralité d'électrodes (153),
dans lequel le signal électrique délivré à la pluralité d'électrodes (153) est un signal électrique répétitif avec au moins un temps de retard, et
**caractérisé en ce que** la tension appliquée à la pluralité d'électrodes (153) se situe dans la plage de 20 Veff à 250 Veff, l'énergie transmise à la pluralité d'électrodes (153) se situe dans la plage de 4,0 W à 625 W, la durée pendant laquelle le signal électrique est conduit à la pluralité d'électrodes (153) se situe dans la plage de 10 ms à 300 ms, et le temps de retard se situe dans la plage de 0,1 ms à 500 ms .

2. Appareil selon la revendication 1, dans lequel la pluralité d'électrodes (153) possède une configuration bipolaire.

3. Appareil selon la revendication 1, dans lequel le générateur de signal électrique (130) est un générateur de signal à haute fréquence, laquelle fréquence se situe dans la plage de 0,1 MHz à 100 MHz.

4. Appareil selon la revendication 1, dans lequel au moins un paramètre parmi une profondeur de pénétration de l'électrode dans la peau cible, la tension appliquée à la pluralité d'électrodes, l'énergie transmise à la pluralité d'électrodes, la durée pendant laquelle un signal électrique est conduit à la pluralité d'électrodes, et le temps de retard pendant lequel un signal électrique n'est pas conduit à la pluralité d'électrodes, est fixé au préalable.

5. Appareil selon la revendication 1, dans lequel l'appareil comporte un module d'électrodes (150) comprenant un réseau auquel la pluralité d'électrodes (153) est fixée.

6. Appareil selon la revendication 5, dans lequel l'appareil comporte une unité motrice (170) pour entraîner l'électrode ou le module d'électrodes afin que l'électrode pénètre dans la peau.
